# NOUVEAU FASCICULE DE BREVET EUROPEEN

(11) **EP 0 776 655 B2**
(45) Date de publication et mention de la décision concernant l'opposition: **15.09.2004**
(45) Mention de la délivrance du brevet: 27.03.2002
(21) Numéro de dépôt: 96402375.8
(22) Date de dépôt: 07.11.1996
(51) Int. Cl.: A61K 7/48, A61K 7/02, A61K 7/50, A61K 7/00

(54) **Utilisation d'un polypholoside dans une composition nettoyante ou démaquillante et composition le comprenant**
Verwendung eines Polyholosides in einer Reinigungs- oder Abschminkzusammensetzung und dieses enthaltende Zusammensetzung
Use of a polyholoside in a cleansing or make-up removing composition and composition containing it

(30) Priorité: 01.12.1995 FR 9514470; 21.05.1996 FR 9606288
(43) Date de publication de la demande: 04.06.1997
(73) Titulaire: L'OREAL, 75008 Paris (FR)
(72) Inventeur: Carrel, Marie-Laure, 75007 Paris (FR); Bui-Bertrand, Lien, 91630 Savigny/s/Orge (FR)
(74) Mandataire: Dodin, Catherine

(56) Documents cités:
- EP-A- 0 285 829
- EP-A- 0 569 591
- EP-A- 0 651 990
- WO-A-92/06778
- FR-A- 2 068 447
- FR-A- 2 698 102
- JP-A- 6 485 905
- US-A- 3 240 775
- JF Molina Kosmetik Jahrbuch 1995, pp. 319-323, Verlag für Chemische Industrie, Augsburg
- Manufacturing Chemist, Juin 1983, page 33 (extrait d'article)
- Manufacturing Chemist, juin 2003, page 20(extrait d'article)
- JF Molina "Dalla biogtechnologia un nuovo polisaccaride a doppia attivita", Cosmetics & Toiletries Ed.it., 1995, vol.1, pp. 26-29
- EJ Masters "Cleansing creams and lotions", Cosmetics, Science and technology, Wiley Interscience, 1972, pp. 1-2
- E. Charlet "Kosmetik für Apotheker", 1989, Wissenschaftlich Verlagsgesellschaft, Stuttgart
- T. Miyazaki "Structure and physiological activity of polysaccharides (traduction en Anglais d'une partie), 20 janvier 1990
- "Biochemical organic compounds - Diagnostic reagents", Sigma, USA
- G. Proserpio et al "Elementi di fitocosmesi" Juin 1982, Sepem, Milano
- FS D'Amelio "Botanicals : a phytocosmetic desk reference", 1999, CRC Press, Boca Raton
- Collins dictionary, English to German part, page 396, Harper & Collins, 1995
- Pons Globalwörterbuch, Weis-Mattutat Französich-Deutsch, p 815, 1987
- Römpp Chemie Lexikon, 9ème édition élargie, p 4990, Georg Thieme Verlag, Stuttgart, 1992
- Kosmetik : Entwicklung, Herstellungund Anwendung kosmetischer Mittel, Wilfried Umbach, pp. 61-63 et 223-229, Georg Thieme Verlag, Stuttgart, 1988
- Römpp Chemie Lexikon, 9ème édition élargie, pp. 1745-1746, Georg Thieme Verlag, Stuttgart, 1990
- Römpp Chemie Lexikon, 9ème édition élargie, pp. 95-96, Georg Thieme Verlag, Stuttgart, 1990
- Evaluation of synthetic oily materials as bases for lipsticks, cleansing milks and foundation emulsions

## Description

La présente invention se rapporte à l'utilisation d'un polyholoside dans une composition notamment cosmétique destinée au nettoyage et/ou au démaquillage de la peau et/ou des muqueuses et/ou des yeux.

Il est connu d'utiliser des huiles démaquillantes dans des compositions pour le démaquillage de la peau et/ou des yeux. Généralement, ces compositions comprennent également des tensioactifs qui permettent d'améliorer l'élimination de ladite huile et/ou des traces de maquillage. Or, on sait que l'utilisation de ces compositions peut laisser une impression d'inconfort, notamment sur les paupières et/ou sur les yeux.
Il est également connu des compositions de démaquillage à base uniquement de tensioactifs, qui vont permettre l'élimination des traces de maquillage tout en facilitant la mise en émulsion de la composition, en rendant compatible les phases grasse et aqueuse de la composition lorsqu'elle se présente sous la forme d'une émulsion. Toutefois, les tensioactifs peuvent présenter l'inconvénient d'être plus ou moins irritants pour la peau

Le document WO-A-92/16778 décrit l'utilisation d'alkylpolyosides et notamment d'alkylpolyglucosides comme émulsionnants pour la préparation d'émulsions.
Le document EP-651990 décrit une composition démaquillante comprenant des esters gras en C₁₂ à C₂₀.

L'invention a pour but de pallier les inconvénients de l'art antérieur et de proposer une composition qui permet un nettoyage et/ou un démaquillage facile et adéquat, qui n'est pas irritante pour la peau et/ou les yeux, et qui ne contient pas de tensioactif.

Un objet de l'invention est une composition pour le nettoyage et/ou le démaquillage de la peau et/ou des muqueuses et/ou des yeux, comprenant au moins un polyholoside hétérogène choisi parmi les polyholosides hétérogènes comprenant au moins des motifs fucose, galactose et acide galacturonique et/ou les polyholosides de type alginate, et comprenant une phrase huileuse qui comprend un ester d'acide gras, obtenu à partir d'un alcool à chaîne droite ou ramifiée ayant de 1 à 17 atomes de carbone et d'un acide gras à chaîne droite ou ramifiée ayant de 3 à 18 atomes de carbone.

Un autre objet de l'invention est un procédé de nettoyage et/ou de démaquillage de la peau et/ou des muqueuses et/ou des yeux, caractérisé en ce qu'on applique sur la peau et/ou sur les muqueuses et/ou sur les yeux une composition telle que définie ci-dessus.

On a en effet constaté que l'utilisation d'un polyholoside selon l'invention permet d'obtenir une composition nettoyante et/ou démaquillante peu ou non irritante et peu collante, qui présente de surcroît un toucher doux et agréable.

Un autre avantage procuré par l'utilisation d'un tel polyholoside est d'améliorer la stabilisation de la composition finale, lorsqu'elle se présente sous forme d'émulsion, grâce aux propriétés auto-émulsionrantes du polyholoside employé, en particulier lorsqu'il s'agit d'un polyholoside comprend des motifs fucose, galactose et acide galacturonique. De plus, l'incorporation d'un polyholoside dans des compositions, notamment cosmétiques, permet l'obtention d'une composition gélifiée sans ajout supplémentaire d'agent gélifiant classiquement utilisé. Le gel obtenu est lisse et onctueux.

Les saccharides, de formule Cₙ(H₂O)ₙ, sont généralement divisés en deux catégories : les oses ou sucres simples, et les osides ou association de plusieurs molécules.
Parmi les osides, on peut distinguer (1) les holosides qui sont formés uniquement de sucres et (2) les hétérosides qui sont constitués par un ou plusieurs oses et une partie non glucidique.
De plus, parmi les polyholosides, on peut encore distinguer les polyholosides homogènes qui résultent de l'association d'un même ose, et les polyholosides hétérogènes qui résultent soit de l'association d'oses différents, soit de l'association d'oses ayant la même formule chimique brute mais de configuration géométrique différente (isomères D et L par exemple), considérés également dans la présente description comme des oses différents.
C'est cette dernière catégorie, constituée des polyholosides hétérogènes, qui est plus particulièrement concernée par la présente invention.

Dans le cadre de la présente invention, on peut utiliser un polyholoside hétérogène seul, ou un mélange de polyholosides hétérogènes.

Le polyholoside selon l'invention peut être un alginate (poly mannuronate et guluronate) tel qu'un alginate de sodium, un alginate de propylène glycol, un alginate de calcium, ou un alginate de glycéryle.

Le polyholoside selon l'invention peut être un polyholoside comprenant des motifs fucose, galactose et acide galacturonique, et, par exemple peut comprendre un enchaînement linéaire de α-L-Fucose, de α-D-Galactose et d'acide galacturonique. Dans ce cas, il présente de préférence une viscosité de 800-1200 mPa.s (viscosité Brookfield LV31, 12 tours/min, à 30°C) lorsqu'il est mis en solution dans l'eau à une concentration d'environ 1 % en poids.
Le motif fucose peut être présent en une quantité de 10-90% en poids, de préférence 15-35% en poids, par rapport au poids de matière sèche de polyholoside.

Les polyholosides selon l'invention sont de préférence introduits dans la composition sous forme de solution aqueuse qui peut comprendre 0,1 à 5% en poids de polyholoside.
Le polyholoside peut être présent dans la composition finale en une quantité de 0,01 à 5% en poids. Lorsque le polyholoside comprend un enchaînement linéaire de α-L-Fucose, de α-D-Galactose et d'acide galacturonique, il est de préférence présent dans la composition à raison de 0,01 à 1% en poids.

Les polyholosides selon l'invention peuvent donc être utilisés en tant qu'agent démaquillant, en particulier dans une composition pour le nettoyage et/ou démaquillage de la peau du corps ou du visage, des muqueuses telles que les lèvres, et/ou des yeux.
Ladite composition peut se présenter sous la forme d'une émulsion, notamment huile-dans-eau ou eau-dans-huile, voire sous la forme d'une émulsion multiple. Elle peut également se présenter sous forme d'une lotion, par exemple biphasée, d'une crème, d'un lait, voire d'une mousse.

La phrase huileuse de la composition selon l'invention peut comprendre une huile animale, végétale, minérale, siliconée, fluorée et/ou synthétique.
La phase huileuse peut également comprendre des alcools gras ou des acides gras, ainsi que des tensioactifs.

On peut notamment citer les huiles hydrocarbonées telles que l'huile de paraffine ou de vaseline; le perhydrasqualène; l'huile d'arachide; l'huile d'amande douce, de calophyllum, de palme, de ricin, d'avocat, de jojoba, d'olive ou de germes de céréales; des alcools tels que l'alcool oléique, l'alcool linoléique ou linolénique, l'alcool isostéarique ou l'octyl dodécanol. On peut également citer les huiles siliconées telles que les PDMS, éventuellement phénylées telles que les phényltriméthicones.
L'ester d'acides gras peut en particulier être choisi dans le groupe constitué par l'adipate de dioctyle, le palmitate d'éthyl-2 hexyle, l'adipate de diisopropyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle.
La phase huileuse peut être présente à raison de 5-95% en poids dans le cas d'une émulsion.

La composition selon l'invention peut comprendre, en outre,
- un agent permettant la mise en suspension de la phase grasse, par exemple un copolymère d'acrylates d'alkyle en C₁₀-C₃₀ et d'acide acrylique ou méthacrylique ou de leur ester (Pemulen TR1, Pemulen TR2, Carbopol 1342 de GOODRICH); ou un copolymère acrylamide/acide méthylpropane sulfonique (Sepigel de SEPPIC), et/ou
- un agent de mise en dispersion de la phase grasse, tel qu'un système émulsionnant ou vésiculaire à base de vésicules, éventuellement de taille nanométrique, constituées de lipides ioniques ou non ioniques, et en particulier les systèmes émulsionnants bien connus de l'homme du métier constitués de stéarate de glycéryl/PEG 100 stéarate (CTFA), d'alcool cétylique et d'alcool stéarylique.

La composition de l'invention peut comprendre, en outre, un agent pour modifier sa viscosité, et obtenir des textures plus ou moins gélifiées, tel que :
- les dérivés cellulosiques (carboxyméthylcellulose, hydroxyéthylcellulose, hydroxypropylméthylcellulose),
- les gommes naturelles telles que les gommes de xanthane, de guar, de caroube, les scléroglucanes, les dérivés de chitine ou de chitosane, les carraghénanes,
- les dérivés polycarboxyvinyliques du type Carbomer (vendues par GOODRICH sous les dénominations Carbopol, 940, 951, 980, ou par 3V-SIGMA sous la dénomination Synthalen K ou Synthalen L).

La composition selon l'invention peut également comprendre, de façon connue, des adjuvants couramment utilisés dans le domaine considéré, tels que les conservateurs, les antioxydants, les parfums, les charges telles que le kaolin ou l'amidon, voire les microsphères creuses, les pigments, les filtres UV, les séquestrants, les huiles essentielles, les matières colorantes, les actifs hydrophiles ou lipophiles tels que les hydratants, notamment la glycérine, le butylène glycol, les anti-inflammatoires comme l'allantoïne, le bisabolol, les anti-radicaux libres comme la vitamine E ou ses dérivés, les apaisants tels que l'eau de bleuet, l'extrait d'iris, les dépigmentants, les actifs biologiques tels que l'urée, les acides aminés, les vitamines et leurs dérivés, les protéines, l'acide salicylique et ses dérivés, les α-hydroxyacides, l'acide pyrrolidone carboxylique et ses sels, les céramides.
Bien entendu l'homme du métierveillera à choisirce ou ces éventuels composés complémentaires, et/ou leur quantité, de manière telle que les propriétés avantageuses de la composition selon l'invention ne soient pas, ou substantiellement pas, altérées par l'adjonction envisagée.

La composition présente de préférence un pH qui respecte la peau, généralement compris entre 5 et 8, de préférence un pH de 5,5 à 7,5.

La composition selon l'invention peut être conditionnée dans tout type d'article de conditionnement. En particulier, les compositions de nettoyage et/ou démaquillage selon l'invention peuvent être avantageusement conditionnées en conditionnement mono-dose (ou uni-dose), à savoir un conditionnement tel que son contenu peut être utilisé en totalité lors d'un usage unique.
Un tel conditionnement peut en particulier être obtenu selon l'état de la technique, notamment par extrusion-soufflage-moulage d'une matière plastique.
Ce procédé permet donc à un contenant d'être moulé par soufflage, rempli avec la composition, puis scellé, en une seul opération aseptique et continue.
Dans une première étape, le polymère, notamment sous forme de granulé, peut être extrudé, ce qui permet également de le stériliser. Un dispositif de moulage se présentant sous forme de deux moitiés, peut venir se refermer sur le polymère extrudé pour former le corps du contenant. On peut alors injecter à l'intérieur du moulage un gaz tel que de l'air filtré stérile, ce qui va permettre de 'souffler' le contenant selon la forme du moule. On peut ensuite remplir le conditionnement ainsi obtenu avec la composition de nettoyage selon l'invention, ladite composition étant de manière préférée préalablement stérilisée. Quand le volume requis est rempli, on peutfermer le conditionnement. Un tel procédé est notamment décrit dans l'articule 'Sterillty and versatility in cosmetic cream packaging' de D. Wilson, paru dans Cosmetics and Toiletries Manufacture Worlwide, pages 253-256.
Un autre objet de l'invention est donc une composition pour le nettoyage et/ou le démaquillage de la peau et/ou des muqueuses et/ou des yeux, telle que défine ci-dessus comprenant un polyholoside hétérogène qui comprend un motif fucose présent en une quantité de 10-90% en poids, de préférence 15-35% en poids, par rapport au poids de matière sèche de polyholoside, caractérisée par le fait qu'elle est conditionnée en monodose.

L'invention est illustrée plus en détail dans les exemples suivants.

### Exemple 1: Lotion démaquillante pour les yeux

- FUCOGEL 1000 50 g
- Palmitate d'éthyl-2 hexyle 10 g
- Cyclopentadiméthylslloxane 20 g
- Butylène glycol 5 g
- Conservateur qs
- Eau qsp 100 g Cette lotion, qui ne contient pas de tensioactif, permet un démaquillage efficace et agréable des yeux, même pour un maquillage waterproof.

### Exemple 2: Lait démaquillant

- Palmitate d'octyle 35 g
- Glycérine 2 g
- FUCOGEL 1000 50 g
- Polymère réticulé acrylates C10-C30/alkylacrylates 0,1 g
- Triéthanolamine 0,1 g
- Acides aminés de blé 1 g
- Conservateur qs
- Eau qsp 100 g Le lait obtenu, qui ne contient pas de tensioactif, possède de bonnes propriétés cosmétiques.

### Exemple 3: A titre d'information

On prépare une émulsion huile-dans-eau comprenant 40% en poids d'une huile connue pour ne pas avoir de propriétés démaquillantes, l'huile de vaseline.
On ajoute dans l'émulsion :
- soit un polyholoside selon l'invention (FUCOGEL 1000)
- soit un agent gélifiant n'ayant pas de propriétés démaquillantes (Carbopol 981 de GOODRICH)

Le pouvoir démaquillant des différents émulsions est contrôlé à l'aide de la technique dite du "robot démaquilleur" :
. l'appareil se compose d'une plaque et d'un bras muni d'un poids exerçant sur la plaque une pression de 100 g/cm² et équipé à l'une de ses extrémités d'un coton qui glisse sur la plaque.
. on dépose sur la plaque une fine couche d'un mascara waterproof noir commercialisé sous le nom de Kéracils par Lancôme.
. cette plaque est mise à sécher pendant 4 h.
. on réalise ensuite 9 passages simples sans retour, d'un coton imbibé de 40 gouttes de composition à tester, le coton étant changé après chaque passage.
. on évalue visuellement la quantité de mascara restante sur la plaque après ces neuf passages.
. on note le nombre de cotons nécessaire à un démaquillage parfait. L'absence totale de mascara sur la plaque constitue, selon ce test, un démaquillage parfait.

On obtient les résultats suivants :

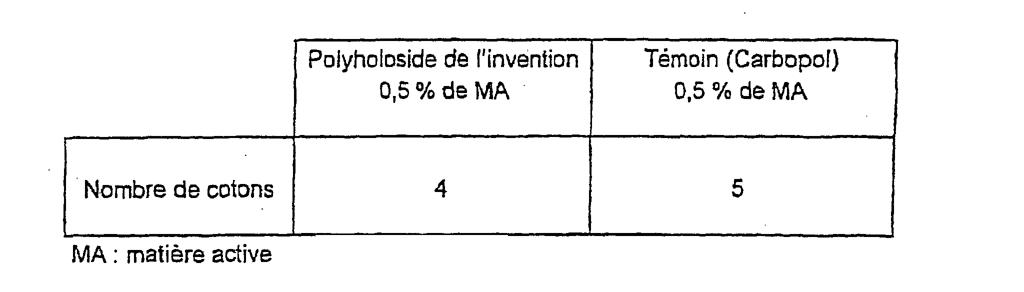

On constate donc qu'à une teneur de 0,5% de matière active, le polyholoside selon l'invention présente un pouvoir démaquillant supérieur à celui du témoin.

### Exemple 4: A titre d'information

On prépare 4 émulsions huile-dans-eau comprenant 40% en poids d'une huile de vaseline et soit un polyholoside selon l'invention (FUCOGEL 1000), solt un agent gélifiant n'ayant pas de propriétés démaquillantes (Carbopol 981).
On les utilise pour démaquiller les yeux de 20 ou 24 personnes préalablement maquillées avec Kéracils de Lancôme.

On obtient les résultats suivants.

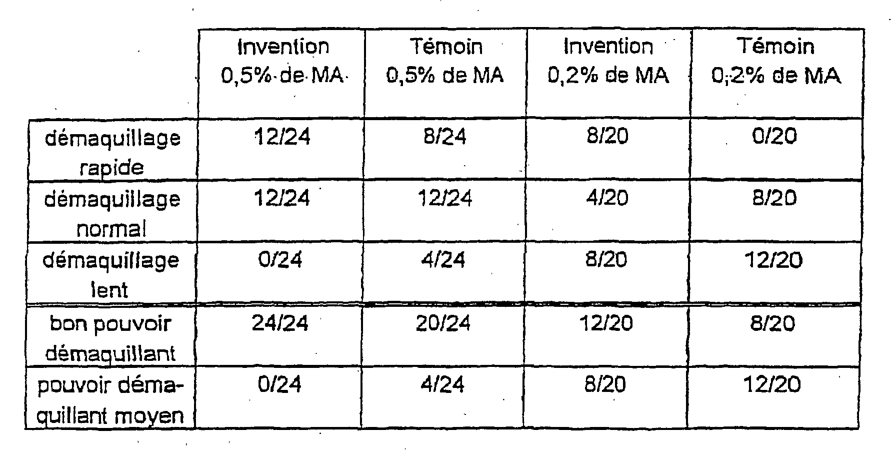

On constate que les réponses obtenues sur la vitesse de démaquillage et le pouvoir démaquillant des compositions selon l'invention sont toujours supérieures aux compositions témoins.

De plus, les paupières démaquillées avec les émulsions selon l'invention sont de toucher et d'aspect moins huileux et moins brillants que celles démaquillées selon l'art antérieur.

### Exemple 5: A titre d'information

On étudie, dans cet exemple, les propriétés démaquillante d'un polyholoside selon l'invention.
On prépare une émulsion huile-dans-eau comprenant 40% en poids d'huile de vaseline. On ajoute du FUCOGEL 1000 et l'on teste la composition obtenue à l'aide du robot-démaquilleur.
On obtient les résultats suivants.

| | | | | |
|---|---|---|---|---|
| Concentration en polyholoside (% MA) | 0,5 % | 0,4% | 0,3% | 0,2% |
| Nombre de cotons | 6 | 7 | 8 | 9 |

### Exemple 6: A titre d'information

On teste le pouvoir démaquillant d'une émulsion huile-dans-eau comprenant 40% en poids d'huile de vaseline et 0,5% en poids d'un gel démaquillant composé d'un mélange de FUCOGEL 1000 et de Carbopol 981, en quantité variable.

On obtient les résultats suivants:

| | | | | |
|---|---|---|---|---|
| FUCOGEL 1000 | 0,2% | 0,25% | 0,3% | 0,4% |
| Carbopol 981 | 0,3% | 0,25% | 0,2% | 0,1% |
| Nombre de cotons | 9 | 8 | 7 | 6 |

## Revendications

1. Composition pour le nettoyage et/ou le démaquillage de la peau et/ou des muqueuses et/ou des yeux, comprenant au moins un polyholoside hétérogène choisi parmi les polyholosides hétérogènes comprenant au moins des motifs fucose, galactose et acide galacturonique, et/ou les polyliolosides de type alginate, et comprenant une phase huileuse qui comprend un ester d'acide gras, obtenu à partir d'un alcool à chaîne droite ou ramifiée ayant de 1 à 17 atomes de carbone et d'un acide gras à chaîne droite ou ramifiée ayant de 3 à 18 atomes de carbone.

2. Composition selon la revendication précédente, dans laquelle l'ester est choisi dans le groupe constitué par l'adipate de dioctyle, le palmitate d'éthyl-2 hexyle, l'adipate de diisopropyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 heayle, le palmitate de méthyle, le myristate de butyle, le myristate d'isobutyle, le palmitate d'éthyle, le laurate d'isohexyle, le laurate d'hexyle, l'isostéarate d'isopropyle.

3. Composition selon l'une des revendications précédents, dans laquelle le polyholoside hétérogène comprend un motif fucose présent en une quantité de 10-90% en poids, de préférence 15-35% en poids, par rapport au poids de matière sèche de polyholoside.

4. Composition selon l'une des revendications précédentes, dans laquelle le polyholoside comprend un enchaînement linéaire de α-L-Fucose, de α-D-Galactose et d'acide galacturonique.

5. Composition selon l'une des revendications précédentes, dans laquelle le polyholoside est présent dans la composition finale en une quantité de 0,01 à 5% en poids, de préférence 0,01 à 1% en poids, par rapport à la composition.

6. Composition selon l'une des revendications précédentes, se présentant sous la forme d'une émulsion, d'une lotion notamment biphasée, d'une crème, d'un lait, d'une mousse.

7. Composition selon l'une des revendications précédentes, **caractérisée par le fait qu'**elle est conditionnée en monodose.

8. Procédé de nettoyage et/ou de démaquillage de la peau et/ou des muqueuses et/ou des yeux, **caractérisé en ce qu'**on applique sur la peau et/ou sur les muqueuses et/ou sur les yeux, une composition de nettoyage et/ou de démaquillage, selon la revendication 1.

## Patentansprüche

1. Zusammensetzung zur Reinigung und/oder zum Abschminken der Haut und/oder der Schleimhäute und/ oder der Augen, die mindestens ein heterogenes Polyholosid enthält, das unter den heterogenen Polyholosiden, die zumindest eine Fucoseeinheit, Galactoseeinheit und Galacturonsäureeinheit enthalten, und/oder den Polyholosiden vom Alginattyp ausgewählt ist, und die eine Ölphase mit einem Fettsäureester enthält, wobei der Fettsäureester aus einem geradkettigen oder verzweigten Fettalkohol mit 1 bis 17 Kohlenstoffatomen und einer geradkettigen oder verzweigten Fettsäure mit 3 bis 18 Kohlenstoffatomen gebildet wird.

2. Zusammensetzung nach dem vorhergehenden Anspruch, wobei der Ester unter Dioctyladipat, 2-Ethylhexylpalmitat, Diisopropyladipat, 2-Ethylhexylhexanoat, Ethyllaurat, Methylmyristat, Octyldodecyloctanoat, Isodecylneopentanoat, Ethylmyristat, Myristylpropionat, 2-Ethylhexyl-2-ethylhexanoat, 2-Ethylhexyloctanoat, 2-Ethylhexylcaprat/caprylat, Methylpalmitat, Butylmyristat, Isobutylmyristat, Ethylpalmitat, Isohexyllaurat, Hexyllaurat und Isopropylisostearat ausgewählt ist.

3. Zusammensetzung nach einem der vorhergehenden Ansprüche, wobei das heterogene Polyholosid Fucoseeinheiten in einer Menge von 10 bis 90 Gew.-% und vorzugsweise 15 bis 35 Gew.-%, bezogen auf die Trockensubstanz des Polyholosids, enthält.

4. Zusammensetzung nach einem der vorhergehenden Ansprüche, wobei das Polyholosid eine gerade Verknüpfung von α-L-Fucose, α-D-Galactose und Galacturonsäure enthält.

5. Zusammensetzung nach einem der vorhergehenden Ansprüche, wobei das Polyholosid in der fertigen Zusammensetzung in einem Mengenanteil von 0,01 bis 5 Gew.-% und vorzugsweise 0,01 bis 1 Gew.-%, bezogen auf die Zusammensetzung, enthalten ist.

6. Zusammensetzung nach einem der vorhergehenden Ansprüche, die in Form einer Emulsion, einer Lotion, insbesondere zweiphasigen Lotion, einer Creme, einer Milch oder eines Schaums vorliegt.

7. Zusammensetzung nach einem der vorhergehenden Ansprüche, **dadurch gekennzeichnet, daß** sie in Form einer Einzeldosis verpackt ist.

8. Verfahren zur Reinigung und/oder zum Abschminken der Haut und/oder der Schleimhäute und/oder der Augen, **dadurch gekennzeichnet, daß** auf die Haut und/oder die Schleimhäute und/oder die Augen eine Zusammensetzung zur Reinigung und/oder zum Abschminken nach Anspruch 1 aufgetragen wird.

## Claims

1. Composition to clean and/or remove make-up from the skin and/or the mucous membranes and/or the eyes, this composition comprising at least one heterogeneous polyholoside chosen from heterogeneous polyholosides comprising at least fucose, galactose and galacturonic acid units, and/or alginate-type polyholosides, and comprising an oily phase which comprises a fatty acid ester, obtained from an alcohol with a straight or branched chain having from 1 to 17 carbon atoms and from a fatty acid with a straight or branched chain having from 3 to 18 carbon atoms.

2. Composition according to the preceding claim, in which the ester is chosen from the group consisting of dioctyl adipate, 2-ethylhexyl palmitate, diisopropyl adipate, 2-ethylhexyl hexanoate, ethyl laurate, methyl myristate, octyldodecyl octanoate, isodecyl neopentanoate, ethyl myristate, myristyl propionate, 2-ethylhexyl 2-ethylhexanoate, 2-ethylhexyl octanoate, 2-ethylhexyl caprate/caprylate, methyl palmitate, butyl myristate, isobutyl myristate, ethyl palmitate, isohexyl laurate, hexyl laurate and isopropyl isostearate.

3. Composition according to either of the preceding claims, in which the heterogeneous polyholoside comprises a fucose unit present in an amount of 10-90% by weight, preferably 15-35% by weight, relative to the weight of polyholoside solids.

4. Composition according to one of the preceding claims, in which the polyholoside comprises a linear sequence of α-L-fucose, α-D-galactose and galacturonic acid.

5. Composition according to one of the preceding claims, in which the polyholoside is present in the final composition in an amount of from 0.01 to 5% by weight, preferably 0.01 to 1% by weight, relative to the composition.

6. Composition according to one of the preceding claims, which is in the form of an emulsion, a lotion, in particular a two-phase lotion, a cream, a milk or a foam.

7. Composition according to one of the preceding claims, **characterized in that** it is packaged in single-dose form.

8. Process for cleaning and/or removing make-up from the skin and/or the mucous membranes and/or the eyes, **characterized in that** a cleaning and/or make-up removing composition according to Claim 1 is applied to the skin and/or to the mucous membranes and/or to the eyes.
